# EUROPEAN PATENT SPECIFICATION

(11) **EP 1 504 753 B1**
(45) Date of publication and mention of the grant of the patent: **20.12.2006**
(21) Application number: 04017205.8
(22) Date of filing: 21.07.2004
(51) Int. Cl.: A61Q 11/00, A61K 8/25, A61K 8/22, A61K 8/34

(54) **Tooth whitening material comprising magnesium sodium silicate and urea peroxide**
Zahnweissmittel enthaltend Natriummagnesiumsilikat und Harnstoffperoxid
Composition pour le blanchissement des dents comprenant du silicate de sodium et magnésium et du péroxyde d'urée

(30) Priority: 08.08.2003 JP 2003290173
(43) Date of publication of application: 09.02.2005
(73) Proprietor: GC Corporation, Itabashi-ku, Tokyo (JP)
(72) Inventor: Mori, Daizaburo GC Corporation, Tokyo (JP); Yamaguchi, Shin GC Corporation, Tokyo (JP); Ikushima, Keisuke GC Corporation, Tokyo (JP)
(74) Representative: Müller-Boré & Partner Patentanwälte

(56) References cited:
- EP-A- 1 457 200
- US-A1- 2002 141 955
- US-A1- 2003 044 360
- DATABASE WPI Section Ch, Week 199822 Derwent Publications Ltd., London, GB; Class A96, AN 1998-243213 XP002305892 & ES 2 113 272 A1 (LAB ERN SA) 16 April 1998 (1998-04-16)
- DATABASE WPI Section Ch, Week 200158 Derwent Publications Ltd., London, GB; Class D21, AN 2001-528109 XP002305893 & KR 2001 025 768 A (OH S G) 6 April 2001 (2001-04-06)
- DATABASE WPI Section Ch, Week 198112 Derwent Publications Ltd., London, GB; Class D25, AN 1981-20712D XP002305894 & SU 689 940 B (DUBOV YA M) 8 October 1979 (1979-10-08)

## Description

The present invention relates to a tooth whitening material used for bleaching a tooth having a coloring substance deposited.

As whiteness of teeth is generally considered as an important cosmetic factor, there are strong demands for whitening teeth mainly in young females, and cases of requesting whitening of teeth are being increased. Whitening of teeth is basically to achromatize and/or remove a pigment deposited on a tooth through a chemical reaction, and such methods are mainly employed that use hydrogen peroxide.

Examples of the methods include such a method in that a bleaching agent formed by mixing silicic anhydride with 35% aqueous hydrogen peroxide is coated on a surface of a living tooth (as described, for example, in JP-A-5-320033), such a method in that a bleaching agent formed by mixing 35% aqueous hydrogen peroxide and orthophosphoric acid is coated on a surface of a tooth (as described, for example, in JP-A-8-143436), and such a method in that a bleaching agent formed by dispersing carbamide peroxide in a matrix material containing carboxymethylene is placed in a tray, which is then disposed to be made in contact with a tooth to be bleached (as described, for example, in JP-A-8-113520).

In order to bleach teeth effectively, it is desired that a tooth bleaching material to act on a tooth surface has pH around neutral. However, a tooth bleaching material used in the conventional method using hydrogen peroxide is in an acidic state because a peroxide inherently has a pH of 5.0 or lower, and thus teeth cannot be effectively bleached. There is also such a problem in that bleach with low pH decalcifies the tooth surface to damage the dentin.

In order to avoid the problems, it has been considered that the tooth bleaching material is previously made to have alkalinity, and it is also preferred from the standpoint of reduction of damages on teeth and efficiency of bleaching. However, in the case where the pH of the tooth bleaching material is previouslymade to be alkalinity, the storage stability of hydrogen peroxide, as the effective component, is significantly lowered, and thus such measure cannot be practiced from the standpoint of stable supply of products to the users.

Whitening mate rials containing a titanium dioxide photocatalyst, which is less affected by pH, as a main component have been proposed in view of the afo.rementioned problems of the whitening materials (as described, for example, in JP-A-11-92351, JP-A-2000-344640 and JP-A-2002-322041). These whitening materials contain, as effective components, for example, titanium dioxide and, depending on necessity, aqueous hydrogen peroxide in a low concentration, and bleach teeth through occurrence of a redox reaction by utilizing the photocatalytic action of the former component. These attain the object since they cause less adverse affections on teeth owing to the fact that bleaching can be effected under neutral or subacid conditions with aqueous hydrogen peroxide in a low concentration. However, in order to apply the whitening materials, such an operation should be repeated in that the teeth are irradiated with light using a special apparatus after coating the materials, until the teeth are bleached. Therefore, the operation cannot be carried out at home by the patient, but such therapy should be repeated in several times in that light irradiation for 5 minutes is repeated in several times within one day in a clinic. As a result, a period of three days to several months is required for bleaching teeth, which brings about such disadvantages in that not only the physician suffers burden in labor, but also the patient suffers burden in time and economy.

A tooth whitening material using aqueous hydrogen peroxide in a low concentration and polyaniline but using no titanium dioxide has also been proposed (as described, for example, in JP-A-2002-293725). However, it has a problem in storage stability in the state where polyaniline is added, and therefore, it has such disadvantage that polyaniline is separately stored, and two pastes should be mixed immediately before use.

Examples of a thickener having been mainly used in the conventional tooth whitening materials using hydrogen peroxide or titanium dioxide include carboxypolymethylene, polyvinylpyrrolidone, hydroxyethyl cellulose or hydroxymethyl cellulose, as well as an inorganic mineral, such as saponite and magnesium sodium lithium silicate. However, in the cases where only these thickeners are used, such problems occur in that upon loading the gel tooth whitening material on an tray and upon fitting the tray in an oral cavity, the tooth whitening material outflows from the tray to the oral cavity. Accordingly, a tooth whitening material resolving the problems has been demanded.

An object of the present invention is to provide such a tooth whitening material that uses urea peroxide containing no water as an effective component instead of aqueous hydrogen peroxide, exerts high efficiency in bleaching teeth with less damages on teeth due to pH, is in the form of single paste, has good storage stability, and exerts high adhesiveness on a tooth surface.

The present inventors have made earnest investigations to solve the problems and to obtain a tooth whitening material in a weakly acidic to weakly alkaline condition, which attains that deterioration in storage stability due to pH during storage is prevented by avoiding addition of water in the formulation of the whitening material using urea peroxide, damages on teeth are suppressed upon use, and the efficiency of bleaching is improved. As a result, it has been found that in the case where magnesium sodium silicate characterized as it is not swelled with a polyhydric alcohol but is swelled with water is used as a thickener in combination with a polyhydric alcohol, such a tooth whitening material can be obtained in that the magnesium sodium silicate functions as an inorganic filler in the tooth whitening material before use as not being swelled with the polyhydric alcohol, and upon once contact with water in an oral cavity, not only it is increased in viscosity to exhibit ideal adhesiveness as a tooth whitening material on a tooth surface, but also the magnesium sodium silicate exhibits alkalinity only upon contact with water, whereby the tooth whitening material having been adjusted to a weakly acidic to neutral condition during storage can be increased in pH only upon application on a tooth surface depending on necessity.

Accordingly, the present invention relates to a tooth whitening material containing 1 to 20% by weight of magnesium sodium silicate, 10 to 25% by weight of urea peroxide, 40 to 88% by weight of a polyhydric alcohol, and 1 to 15% by weight of a thickener capable of being swelled with the polyhydric alcohol.

It is preferred in the present invention that the polyhydric alcohol is at least one selected from glycerin, polyglycerin, such as diglycerin, propylene glycol, dipropylene glycol, sorbitol, mannitol, ethylene glycol, diethylene glycol and polyethylene glycol.

It is also preferred in the present invention that the thickener capable of being swelled with a polyhydric alcohol is at least one selected from sodium cellulose glycolate, sodium alginate, carboxypolymethylene, carboxymethyl cellulose, sodium carboxymethyl cellulose, calcium carboxymethyl cellulose, a methyl vinyl ether-maleic anhydride copolymer, sodium starch glycolate, sodium starch phosphate, sodium polyacrylate, methylcellulose, crystalline cellulose, hydroxypropyl cellulose and polyvinylpyrrolidone.

It is also preferred in the present invention that the tooth whitening material further contains 0.1 to 10 parts by weight of an inorganic thickener other than magnesium sodium silicate per 100 parts by weight of the tooth whitening material.

The tooth whitening material according to the present invention has the following excellent characteristics. It exhibits excellent storage stability without decomposition of urea peroxide owing to the absence of water during storage, and in the case where it is once used in an oral cavity, magnesium sodium silicate is dissolved with water content of saliva to show alkalinity, whereby the pH at the position where it is adhered to a tooth surface can be increased. Accordingly, damages of teeth due to pH are suppressed, and the bleaching function can be improved. Furthermore, the magnesium sodium silicate functions as an inorganic filler before use since it is not swelled with the polyhydric alcohol, but upon once contact with water in an oral cavity, it also functions as a thickener to provide good adhesiveness to the tooth surface.

Magnesium sodium silicate used in the present invention functions as an inorganic filler before use since it is not swelled with the polyhydric alcohol described later. It furthermore functions, upon use of the tooth whitening material, as a thickener through swelling with water upon contact with water content in an oral cavity, such as saliva, whereby the tooth whitening material is thickened. According to the function, it prevents such a phenomenon that the tooth whitening material is diluted with saliva in the oral to elute urea peroxide as an effective component. It still further exhibits such an important function that it exhibits alkalinity only upon contact with water in the oral upon use, whereby the pH of the part to be bleached is increased only upon use of the tooth whitening material.

The mixed amount of the magnesium sodium silicate is 1 to 20% by weight based on the total amount of the tooth whitening material. In the case where it is less than 1% by weight, the aforementioned functions are difficult to be obtained, and in the case where it exceeds 20% by weight, there is such a possibility that the bleaching function is impaired.

Urea peroxide used in the present invention is an effective component for bleaching teeth. There are many other useful substances than urea peroxide that release hydrogen peroxide, which is effective for bleaching teeth, upon contact with water. However, in the case where aqueous hydrogen peroxide or the like, which substantially contains water, is used, the pH of the tooth whitening material is increased before use (during storage) due to contact of magnesium sodium silicate with water contained in aqueous hydrogen peroxide, whereby the storage stability of hydrogen peroxide is deteriorated due to the alkalinity. Therefore, the other substances are not used in the present invention.

Owing to the aforementioned factors, the present invention uses urea peroxide, which is relatively easily handled and has high bleaching function among peroxides containing substantially no water. It is also important in the present invention that the tooth whitening material does not intentionally contain water. The storage stability of urea peroxide contained in the tooth whitening material having been adjusted with the thickener capable of being swelled with a polyhydric alcohol can be significantly improved owing to the absence of water.

The mixing amount of the urea peroxide used in the tooth whitening material of the present invention is 10 to 25% by weight based on the total amount. In the case where it is less than 10% by weight, it is not preferred since there is such a possibility that the effect of bleaching teeth is lowered, and in the case where it exceeds 25% by weight, it is also not preferred since the whitening material is difficult to be used due to adverse affect on human bodies.

The polyhydric alcohol used in the present invention is a base material of the tooth whitening material and has such a function that urea peroxide as an effective component is certainly adhered on teeth through mixing with the thickener capable of being swelled with a polyhydric alcohol described later. The polyhydric alcohol is preferably at least one selected from glycerin, polyglycerin such as diglycerin, propylene glycol, dipropylene glycol, sorbitol, mannitol, ethylene glycol, diethylene glycol and polyethylene glycol from the standpoint of practicability.

The mixing amount of the polyhydric alcohol is from 40 to 88% by weight based on the total amount of the tooth whitening material. In the case where it is less than 40% by weight, there is such a tendency that the thickener capable of being swelled with a polyhydric alcohol cannot be sufficiently mixed in the tooth whitening material, and in the case where it exceeds 88% by weight, there is such a possibility that the bleaching function of teeth is impaired.

The thickener capable of being swelled with a polyhydric alcohol is added to assist adhesion of the tooth whitening material onto the tooth surface. The thickener is demanded to have a nature of being swelled with a polyhydric alcohol as a solvent of the tooth whitening material for the function that the tooth whitening material is formed into a gel state to promote adhesion on the tooth surface.

The thickener capable of being swelled with a polyhydric alcohol is not specifically limited if only it is a substance capable of gelating the tooth whitening materials and is preferably at least one selected from sodium cellulose glycolate, sodium alginate, carboxypolymethylene, carboxymethyl cellulose, sodium carboxymethyl cellulose, calcium carboxymethyl cellulose, a methyl vinyl ether-maleic anhydride copolymer, sodium starch glycolate, sodium starch phosphate, sodium polyacrylate, methylcellulose, crystalline cellulose, hydroxypropyl cellulose and polyvinylpyrrolidone since these are suitable for use in an oral cavity.

The mixing amount of the thickener capable of being swelled with a polyhydric alcohol is 1 to 15% by weight while it may appropriately adjusted depending on the substances thereof. In the case where it is less than 1% by weight, there is such a tendency that the tooth whitening material cannot be a sufficient gel state, and in the case where it exceeds 15% by weight, there is such a tendency that the adhesiveness to the tooth surface is adversely lowered.

In order to adjust thickening, the tooth whitening material according to the present invention may contain an inorganic thickener other than magnesium sodium silicate thickener in 0.1 to 10 parts by weight per 100 parts by weight of the tooth whitening material consisting of thickener capable of being swelled with magnesium sodium silicate, urea peroxide, polyhydric alcohol. The inorganic thickener may be those having conventionally used in tooth whitening materials, and examples thereof include calcium carbonate, calcium silicate, magnesium silicate, silica powder, various kinds of glass, amorphous hydrous silicic acid, fumed silica and titanium dioxide.

The tooth whitening material according to the present invention may further contain, in addition to the aforementioned components, a flavor, a coloring material, a stabilizer and a solvent containing no water.

A method for bleaching discolored teeth with the tooth whitening material according to the present invention is generally carried out by coating the tooth whitening material of the present invention on the surface of teeth or by placing the tooth whitening material in a dedicated tray, followed by attaching to the teeth. The period of time required for bleaching is generally several minutes to several hours, and after the application of the tooth whitening material to teeth, the same operation may be repeated with a suitable interval until the demanded effect is obtained.

The tooth whitening material according to the present invention is in a form of a single paste, and therefore, there is no necessity of mixing two or more kinds of pastes (including liquids and powder) for use. Furthermore, it requires no irradiation of light, whereby it is not necessary that such therapy is continued for three days to several months in that light irradiation for several minutes is repeated in several times within one day in a clinic, and furthermore, bleach of teeth can be carried out at home in good time of the patient.

The present invention will be described in detail with reference to the following examples.

### EXAMPLE

### Production of Tooth Whitening Material

As shown in Table 1 below, urea peroxide and magnesium sodium silicate powder were added under stirring to one kind or two or more kinds of polyethylene glycol (weight average molecular weight: 400), ethylene glycol, propylene glycol and glycerin as a polyhydric alcohol, followed by stirring. Furthermore, at least one of polyvinylpyrrolidone, carboxypolymethylene (Carbopol, a trade name, produced by NIKKO CHEMICAL Co., Ltd.) and a methyl vinyl ether-maleic anhydride copolymer was added under stirring thereto, followed by stirring. Moreover, depending on necessity, one kind or two or more kinds of silica fine powder (Aerosil R972 and Aerosil OX50, trade names, produced by Nippon Aerosil Co., Ltd.) or titanium dioxide was added thereto, and a flavor (Tooth Paste Flavor, a trade name, produced by Takasago International Corp.), and sodium fluoride and potassium nitrate for reinforcing dentin were added depending on necessity.

### Evaluation of Adhesiveness to Tooth Surface

(1) A tray for tooth whitening was produced so as to accommodate with teeth of a subject.
(2) The tooth whitening materials of the examples and the comparative examples were filled in the tray, and the tray was fit on teeth in an oral.
(3) The state after lapsing two hours after fitting was observed, and the evaluation of the adhesiveness on a tooth surface was made based on the following standard. The results are shown in Table 1.
   A: No outflow of the tooth whitening material from the tray was observed.
   B: Slight outflow of the tooth whitening material from the tray was observed.
   D: A large amount of outflow of the tooth whitening material due to saliva was observed.

### Evaluation of pH Value on Use

(1) 5 g of the tooth whitening materials of the examples and the comparative examples were coated on a bottom of a beaker with a spatula.
(2) 5 g of distilled water was added thereto, and after lapsing 10 minutes, pH of the part of the tooth whitening material was measured after lightly discharging water.
(3) The evaluation of the pH value on use was made based on the following standard based on the pH value thus measured. The results are shown in Table 1.
   A: pH was 6.5 to 7.5
   B: pH was 6.0 or more but less than 6.5.
   C: pH was 5.5 or more but less than 6.0.
   D: pH was less than 5.5.

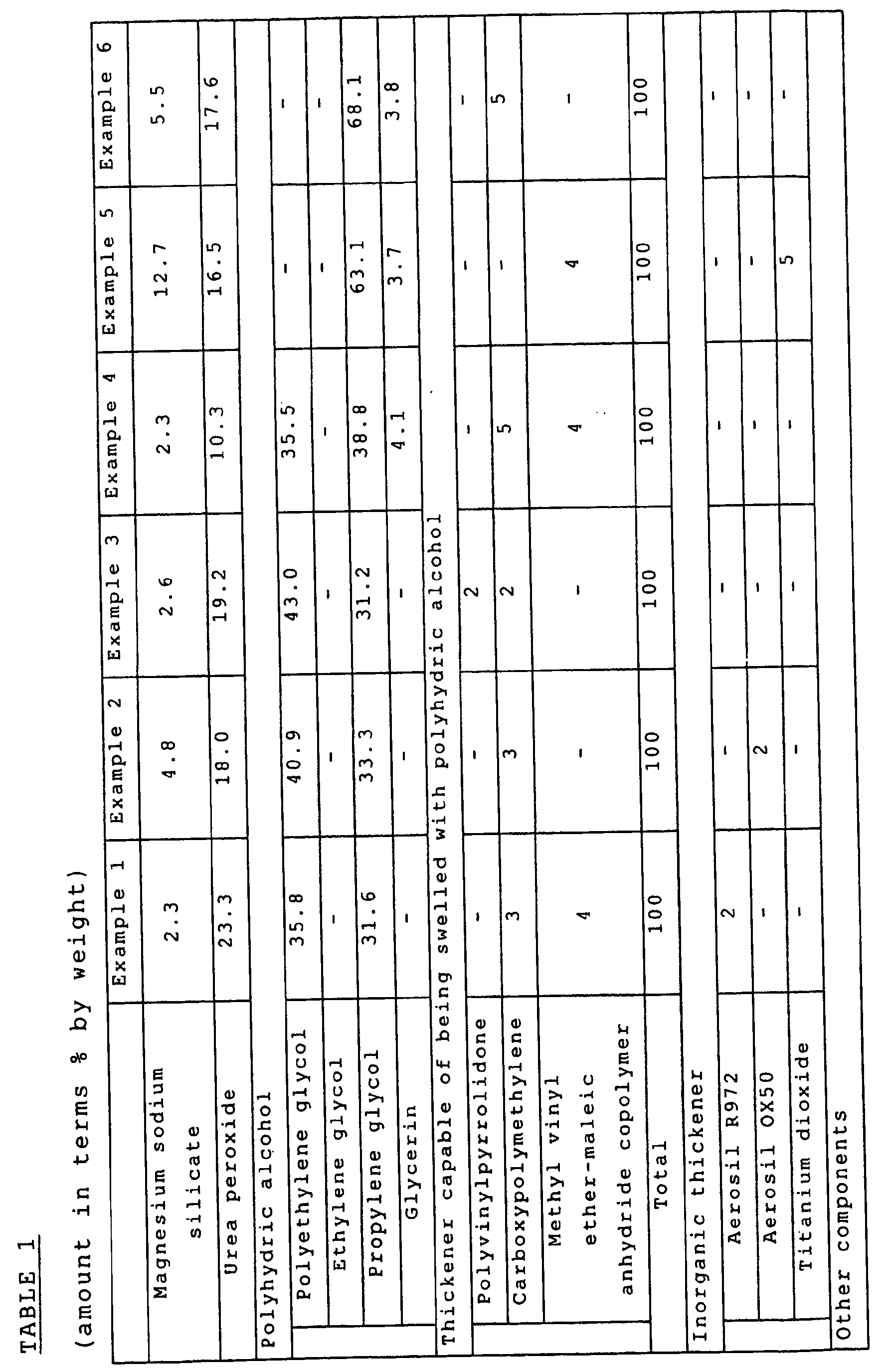

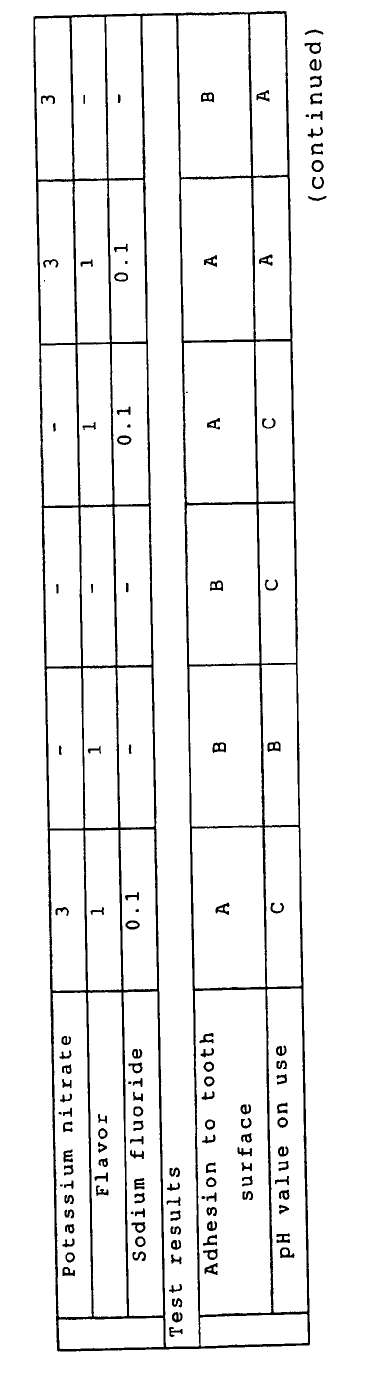

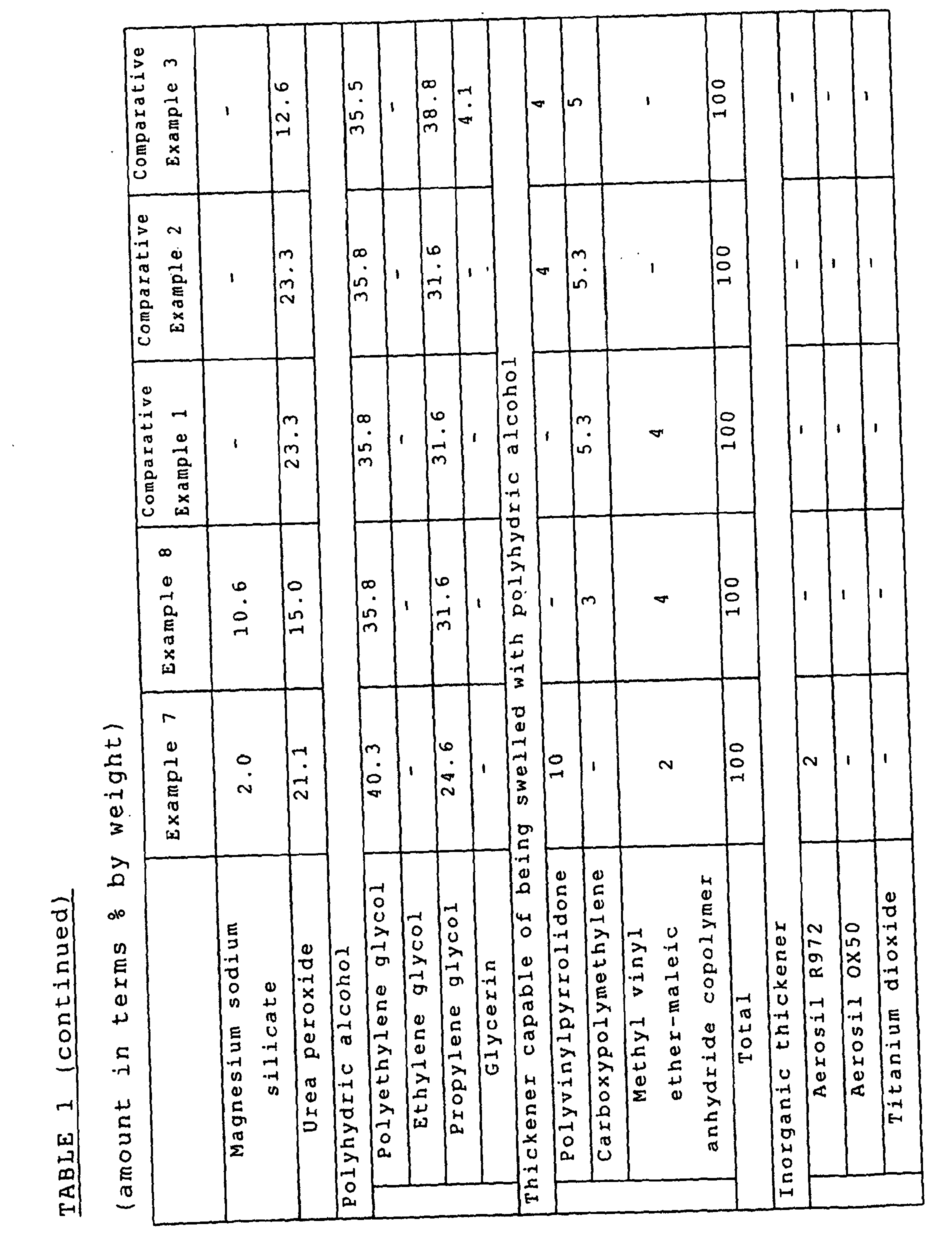

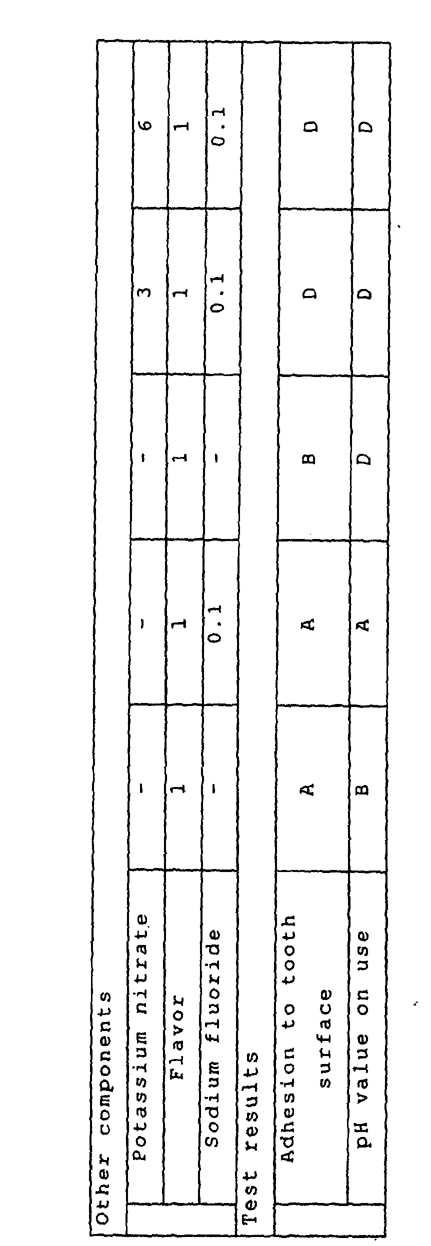

## Claims

1. A tooth whitening material comprising 1 to 20% by weight of magnesium sodium silicate, 10 to 25% by weight of urea peroxide, 40 to 88% by weight of a polyhydric alcohol, and 1 to 15% by weight of a thickener capable of being swelled with the polyhydric alcohol.

2. A tooth whitening material as claimed in claim 1, wherein the polyhydric alcohol is at least one selected from glycerin, polyglycerin, propylene glycol, dipropylene glycol, sorbitol, mannitol, ethylene glycol, diethylene glycol and polyethylene glycol.

3. A tooth whitening material as claimed in claim 1 or 2, wherein the thickener capable of being swelled with a polyhydric alcohol is at least one selected from sodium cellulose glycolate, sodium alginate, carboxypolymethylene, carboxymethyl cellulose, sodium carboxymethyl cellulose, calcium carboxymethyl cellulose, a methyl vinyl ether-maleic anhydride copolymer, sodium starch glycolate, sodium starch phosphate, sodium polyacrylate, methylcellulose, crystalline cellulose, hydroxypropyl cellulose and polyvinylpyrrolidone.

4. A tooth whitening material as claimed in any one of claims 1 to 3, wherein the tooth whitening material further contains 0.1 to 10 parts by weight of an inorganic thickener other than magnesium sodium silicate per 100 parts by weight of the tooth whitening material.

## Patentansprüche

1. Zahnweissmaterial, umfassend 1 bis 20 Gew.-% Natriummagnesiumsilikat, 10 bis 25 Gew.-% Harnstoffperoxid, 40 bis 88 Gew.-% eines mehrwertigen Alkohols und 1 bis 15 Gew.-% eines Verdickungsmittel, das befähigt ist, mit dem mehrwertigen Alkohol gequollen zu werden.

2. Zahnweissmaterial gemäß Anspruch 1, wobei der mehrwertige Alkohol mindestens einer ist, ausgewählt aus Glycerin, Polyglycerin, Propylenglykol, Dipropylenglykol, Sorbitol, Mannitol, Ethylenglykol, Diethylenglykol und Polyethylenglykol.

3. Zahnweissmaterial gemäß Anspruch 1 oder 2, wobei das Verdickungsmittel, welches befähigt ist, mit einem mehrwertigen Alkohol gequollen zu werden, mindestens eines ist, ausgewählt aus Natriumcelluloseglykolat, Nariumalginat, Carboxypolymethylen, Carboxymethylcellulose, Natriumcarboxymethylcellulose, Calciumcarboxymethylcellulose, einem Methylvinylether-Maleinsäureanhydrid-Copolymer, Natriumstärkeglykolat, Natriumstärkephosphat, Natriumpolyacrylat, Methylcellulose, kristalliner Cellulose, Hydroxypropylcellulose und Polyvinylpyrrolidon.

4. Zahnweissmaterial gemäß einem der Ansprüche 1 bis 3, wobei das Zahnweissmaterial weiter 0,1 bis 10 Gew.-Teile eines anorganischen Verdickungsmittels, das von Natriummagnesiumsilikat verschieden ist, pro 100 Gew.- Teilen des Zahnweissmaterials enthält.

## Revendications

1. Substance pour le blanchiment des dents comprenant 1 à 20 % en poids de silicate de sodium et de magnésium, 10 à 25 % en poids de peroxyde d'urée, 40 à 88 % en poids d'un polyol et 1 à 15 % en poids d'un épaississant pouvant gonfler avec le polyol.

2. Substance pour le blanchiment des dents selon la revendication 1, dans laquelle le polyol est au moins un polyol choisi parmi le glycérol, le polyglycérol, le propylène glycol, le dipropylène glycol, le sorbitol, le mannitol, l'éthylène glycol, le diéthylène glycol et le polyéthylène glycol.

3. Substance pour le blanchiment des dents selon la revendication 1 ou 2, dans laquelle l'épaississant pouvant gonfler avec un polyol est au moins un épaississant choisi parmi le glycolate de cellulose sodique, l'alginate de sodium, le carboxypolyméthylène, la carboxyméthylcellulose, la carboxyméthylcellulose sodique, la carboxyméthylcellulose calcique, un copolymère d'éther vinylique et d'anhydride maléique, le glycolate d'amidon sodique, le phosphate d'amidon sodique, le polyacrylate de sodium, la méthylcellulose, la cellulose cristalline, l'hydroxypropylcellulose et la polyvinylpyrrolidone.

4. Substance pour le blanchiment des dents selon l'une quelconque des revendications 1 à 3, dans laquelle la substance pour le blanchiment des dents contient en outre 0,1 à 10 parties en poids d'un épaississant inorganique autre que le silicate de sodium et de magnésium pour 100 parties en poids de la substance pour le blanchiment des dents.
